Europäisches Patentamt

European Patent Office

Office européen des brevets

(11) Publication number: **0 206 332**
**A2**

# EUROPEAN PATENT APPLICATION

(21) Application number: 86108672.6

(51) Int. Cl.⁴: **A 61 B 17/22**

(22) Date of filing: 25.06.86

(30) Priority: 26.06.85 JP 137998/85

(43) Date of publication of application: 30.12.86
Bulletin 86/52

(84) Designated Contracting States: **AT BE CH DE FR GB IT LI LU NL SE**

(71) Applicant: YACHIYODA SANGYO CO., LTD., 2-12-15,
Shinbashi Minato-ku, Tokyo (JP)
Applicant: YACHIYODA KOGYO CO., LTD., No. 671,
Takayanagi Shounan-machi, Higashikatsushika-gun
Chiba-ken (JP)

(72) Inventor: Takayama, Kazuyoshi, No. 4-179, Aza
Obatayama Moniwa, Sendai-shi Miyagi-ken (JP)
Inventor: Kuwahara, Masaaki, No. 9-11, 6-chome
Chomeigaoka, Izumi-shi Miyagi-ken (JP)
Inventor: Kimura, Shuzo, No. 13-10, 2-chome Suwada,
Ichikawa-shi Chiba-ken (JP)

(74) Representative: Schmidt-Evers, Jürgen, Dipl.-Ing. et al,
Patentanwälte Dipl.-Ing. H. Mitscherlich Dipl.-Ing. K.
Gunschmann Dipl.-Ing. Dr.rer.nat. W. Körber Dipl.-Ing. J.
Schmidt-Evers Dipl.-Ing. W. Melzer Postfach 26 01 32,
D-8000 München 26 (DE)

(54) Apparatus for generating high pressure by underwater shock wave.

(57) There is disclosed an apparatus for generating a high pressure by underwater shock wave of the type that it is provided with a shock wave generation chamber (1) of which an inner surface is formed into a shape of a part of a pseudo-ellipsoid of rotation and which is to be filled with liquid and a shock wave generation source (3, 4, 5) for generating shock wave at a first focus (2) of the pseudo-ellipsoid so that a high pressure may be applied by the shock wave to an object set in position at a second focus (9) of the pseudo-ellipsoid of rotation. To avoid contamination and other damage of said object the shock wave generation source comprises a laser means (5), an optical fiber member (4) and a focusing lens (3) for focusing a laser beam on the foregoing focus position (2).

June 25, 1986

APPARATUS FOR GENERATING HIGH PRESSURE BY UNDERWATER
SHOCK WAVE

This invention relates to an apparatus for generating
such a high pressure caused by underwater shock wave
that is applied to a calculus or calculi in a human
body or to other desired object.

Hitherto, as for the apparatus of this kind, there has
been known, for instance, such a type one that is provided with a shock wave generation chamber of which an
inner surface is formed into a shape of a part of a
pseudo-ellipsoid of rotation and such a liquid tank for
dipping a human body that is in communication with an
opening portion of the shock wave generation chamber and
contains a second focus of the pseudo-ellipsoid of rotation therein so that a spark discharge or a microexplosion of a microexplosive may be effected at a position
coincided with the first focus of the pseudo-ellipsoid of
rotation and by this shock wave a high pressure may be
generated and applied to such a calculus in a human body
dipped in the liquid tank that is set in position at the
second focus of the pseudeo-ellipsoid of rotation in the
liquid so as to disintegrate the calculus.

The foregoing conventional apparatus is defective in
that, in the case of use of the spark discharge, there
is a danger of leaking out a high voltage electric current through the liquid to the human body and, in the case of use of the microexplosion of the microexplosive, there is liable to take place a chemical reaction of the explosive with an object to be applied with the high pressure or the liquid is feared to be contaminated with the explosive.

A purpose of this invention is to provide an apparatus free from the foregoing defects.

According to this invention, in such a type of apparatus that it is provided with a shock wave generation chamber of which an inner surface is formed into a shape of a part of a pseudo-ellipsoid of rotation and which is to be filled with liquid and a shockwave generation source for generating shock wave at a first focus of the pseudo-ellipsoid so that a high pressure may be applied by the shock wave to an object set in position at a second focus of the pseudo-ellipsoid of rotation, it is characterized in that the shock wave generation source comprises a laser means, an optical fiber member and a focusing lens for focusing a laser beam on the foregoing focus position.

Now, embodying examples of this invention will be explained with reference to the accompanying drawing as follows:

Fig. 1 is a sectional view of one embodying example of this invention applied to a disintegration apparatus for disintegrating a calculus contained in a human body,

Fig. 2(A) is a sectional view of another embodying example of this invention applied to a coating apparatus,

Fig. 2(B) is a sectional view of a micropiece applied with a coating material.

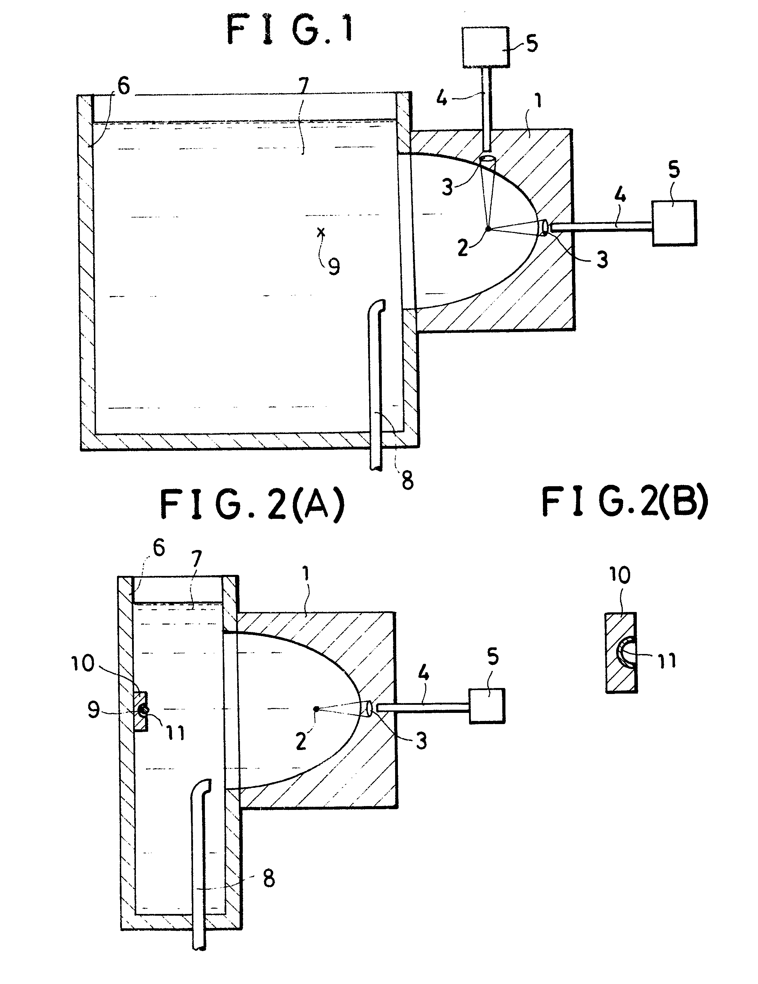

Fig. 1 shows a sectional view of one example of this invention adapted for a calculus disintegration apparatus for disintegrating calculus or calculi in a human body.

Referring to Fig. 1, numeral 1 denotes a shock wave generation chamber of which an inner surface is formed into a shape of a part of a pseudo-ellipsoid of rotation, numeral2 denotes a first focus position of the pseudo-ellipsoid of rotation in the chamber 1, numeral 3 denotes a focusing lens for focusing a laser beam outputted from one end of an optical fiber member 4 on the first focus position 2, and numeral 5 denotes a laser means such as a ruby laser with a Q switch or a Y A G laser with a Q switch connected to the other end of the optical fiber member 4.

The shock wave generation chamber 1 is so attached to the liquid tank 6 that an opening portion thereof may be in communication with an opening portion made in a side wall of the liquid tank 6 for dipping a human body.

In addition, as shown in Fig. 1, there is provided with a water flow jet for removing or sweeping bubbles adhered to an inner surface of the shock wave generation chamber 1 in such a manner that one end thereof may direct toward and along the inner surface of the chamber 1 and the other end thereof is connected to a liquid tank not shown in order that the bubbles adhered to the inner surface of the chamber may be removed or swept by a jet stream of the liquid jetted from the water flow jet 8, lest the shock wave should be interfered with the bubbles.

Next, an operation of the foregoing example will be explained as follows:

A human body is placed in the liquid tank 6 and is so set in position that a part including a calculus in the human body may be coincided with the second focus position of the pseudo-ellipsoid of rotation. Thereafter, two laser means 5, 5 are actuated in sequence or simultaneously and a laser beam in a pulse form from each laser means 5 is focused on the foregoing first position 2 through the optical fiber member 4 and the focusing lens 3. Thus, there is generated shock wave by strong energy of the laser beam at the first focus position 2, and this shock wave is reflected at the inner surface of the shock wave generation chamber 1. This underwater shock wave, unlike a sound wave, when reflected, displays a non-linear type reflection form so called "Mach reflection", and thus it is reflected at a reflection angle which is not equal to the incident angle. The reflected shock wave is focused sharply on the second focus position 9 in the liquid tank 6. Consequently, there is generated a high pressure in sequence or simultaneously at the second focus position 9, so that the calculus is disintegrated.

For instance, when the energy outputted from the foregoing laser means 5 is 15 Joule, there is generated a high pressure as high as 3000 atmospheric pressure at the second focus position 9.

The foregoing example, there are provided two sets of the shock wave generation sources each comprising the laser means 5, the optical fiber member 4 and the focusing lens 3. However, the number of the shock wave generation source is not limited to two, but any desired number thereof is used optionally.

This invention high pressure generation apparatus may be used for various applications. For instance, besides the above application for disintegration of calculus in the human body, it is applicable to a coating apparatus as shown in Fig. 2(A). Namely, a coating material 11 provided in a recessed portion of a micropiece 10 is set to be coincided with the second focus position 9. Under this condition, if there is generated shock wave at the first focus position 2 of the shock wave generation chamber 1 of which an inner surface is formed into a shape of a part of the pseudo-ellipsoid of rotation, in almost the same manner as in the foregoing example, there is generated at the second focus position 9 a high pressure by which the coating material 11 can be firmly coated uniformly on the inner surface of the recessed portion of the micropiece 10, as shown in Fig. 2(B).

It can be considered as another embodying example of this invention, for instance, that any metal and any chemicals which are susceptible to a thermal influence and are liable to undergo a chemical change are mixed and a mixture thereof is placed at the second focus position and is applied with a high pressure caused by the shock wave, so that a new substance can be created.

Thus, according to this invention, a shock wave generation source comprises a laser means, an optical fiber member and a focusing lens for focusing a laser beam on the first focusing position of a pseudo-ellipsoid of rotation in a shock wave generation chamber, so that there is no danger of electric shock and it can be avoided that the object to be applied with a high pressure is chemically reacted with the microexplosive and that the liquid in the tank for transmitting the shock wave is contaminated with the microexplosive as occurred in the conventional apparatus.

CLAIMS

1. An apparatus for generating a high pressure by underwater shock wave of the type that it is provided with a shock wave generation chamber (1) of which an inner surface is formed into a shape of a part of a pseudo-ellipsoid of rotation and which is to be filled with liquid and a shock wave generation source (3, 4, 5) for generating shock wave at a first focus (2) of the pseudo-ellipsoid so that a high pressure may be applied by the shock wave to an object set in position at a second focus (9) of the pseudo-ellipsoid of rotation, characterized in that the shock wave generation source comprises a laser means (5), an optical fiber member (4) and a focusing lens (3) for focusing a laser beam on the foregoing focus position (2).

2. Apparatus according to claim 1, characterized in that there are provided at least two shock generation sources (3, 4, 5), the shock waves generated at the common first focus (2) being generated in sequence or simultaneously.

3. Application of the apparatus according to claim 1 or 2 on disintegration of one or more calculi in a human body.

4. Application of the apparatus according to claim 1 or 2 on coating of pieces (10).

5. Application of the apparatus according to claim 1 or 2 on high pressure mixing of chemicals or substances to create a new substance.

# F I G.1

# F I G.2(A)

# F I G.2(B)